# EUROPÄISCHE PATENTSCHRIFT

(11) **EP 1 064 557 B1**
(45) Veröffentlichungstag und Bekanntmachung des Hinweises auf die Patenterteilung: **06.05.2004**
(21) Anmeldenummer: 99968790.8
(22) Anmeldetag: 09.12.1999
(51) Int. Cl.: G01N 33/80

(54) **VERFAHREN ZUM NACHWEIS VON ANTIKÖRPERN ODER ANTIGENEN SOWIE ZUR BLUTGRUPPENBESTIMMUNG**
METHOD FOR DETECTING ANTIBODIES OR ANTIGENS AND FOR DETERMINING BLOOD GROUPS
PROCEDE POUR METTRE EN EVIDENCE LA PRESENCE D'ANTICORPS OU D'ANTIGENES ET POUR DETERMINER DES GROUPES SANGUINS

(30) Priorität: 09.12.1998 DE 19856703
(43) Veröffentlichungstag der Anmeldung: 03.01.2001
(73) Patentinhaber: DEUTSCHES ROTES KREUZ BLUTSPENDEDIENST BADEN-WÜRTTEMBERG GEMEINNÜTZIGE GMBH, 76530 Baden-Baden (DE)
(72) Erfinder: SPINDLER, Jörg, D-69126 Heidelberg (DE)
(74) Vertreter: Mierswa, Klaus
(86) Internationale Anmeldenummer: PCT/EP1999/009721
(87) Internationale Veröffentlichungsnummer: WO 2000/034790

(56) Entgegenhaltungen:
- US-A- 5 460 940
- SPINDLER J H ET AL: "Photometric evaluation of the solid-phase antiglobulin test using length measurement of the absorption curve." VOX SANGUINIS, Bd. 74, Nr. 1, Januar 1998 (1998-01), Seiten 36-41, XP000917815 ISSN: 0042-9007

## Beschreibung

### Technisches Gebiet:

Die Erfindung betrifft ein Verfahren zum Nachweis von Antikörpern und/oder Antigenen in einer Testflüssigkeit sowie zur Blutgruppenbestimmung durch Reaktion mit einem vorgegebenen spezifischen Bindungspartner, wobei das Antigen bzw. der Antikörper oder der spezifische Bindungspartner in der Testflüssigkeit ungebunden oder an einen Träger gebunden ist und wobei bei positiver Antigen-Antikörper-Reaktion ein Agglutinat aus nachzuweisenden Antigenen bzw. Antikörpern, den entsprechenden spezifischen Bindungspartnern und den Trägern gebildet wird, welches optisch als Sedimentationsbild detektierbar ist, gemäß dem Oberbegriff des Anspruchs 1. Die Erfindung betrifft auch ein Mikroreaktionsgefäß zur Durchführung des Verfahrens wie auch weiterhin die Verwendung von Spitzboden-Mikrotiterplatten zur Durchführung eines Tests zum Nachweis von Antikörpern oder Antigenen durch Detektion einer Agglutinationsreaktion sowie zur Blutgruppenbestimmung.

### Stand der Technik:

Menschliche Blutgruppensubstanzen, aber auch eine Vielzahl von anderen in einem Organismus gebildeten Substanzen, werden durch spezifische Antigen-Antikörper-Reaktionen nachgewiesen. Diese Reaktion ist detektierbar über eine Vernetzung, Agglutination, von Antigenen (Ag), Antikörpern (Ak) und bestimmten Trägersubstanzen, die Antigene oder Antikörper tragen, zu einem makroskopisch erfaßbaren Komplex, dem Agglutinat.

Beispiele dafür sind die erythrozytenseitige Blutgruppenbestimmung sowie die Serumgegenprobe. Bei der erstgenannten werden an die Membran des Spendererythrozyten gebundene Blutgruppen-Antigene durch Reaktion mit Antiseren nachgewiesen, die Antikörper als spezifische Bindungspartner enthalten. Die Antikörper sind bekannt (Such-Antikörper), die sich in einer Testflüssigkeit befinden, zum Beispiel Testantikörper-Aufschwemmung, und die unbekannte Erythrozyten (unbekannte Antigene) enthält. Die Agglutination wird unter geeigneten Reaktionsbedingungen durch Reaktion von Antigen und Antikörper hervorgerufen, womit das System ABO und RH und daneben noch eine Vielzahl von Antigenen bestimmt werden kann.

Bei der Serumgegenprobe sind die Erythrozyten bekannt, die bekannte Antigene (Such-Antigene) aufweisen. Die Testflüssigkeit, zum Beispiel Serum, beinhaltet die unbekannten körpereigenen Antikörper (sog.

Isoagglutinine), die durch Reaktion mit den Test-Erythrozyten nachgewiesen werden. Damit kann ebenfalls die Blutgruppe, jedoch nur ABO, bestimmt werden.

Die Detektion von irregulären Antikörpern, meist inkompletter IgG-Typ, erfolgt indirekt über Anti-IgG. In einem ersten Schritt inkubieren Testerythrozyten mit bekanntem Ag-Muster mit Untersuchungsserum. Die Antikörper binden sich an das Antigen (Ag). In einem zweiten Schritt werden die freien Antikörper mittels Waschzentrifuge in drei Waschschritten ausgewaschen. In einem dritten Schritt wird Antihumanglobulin (Anti-IgG/Coombsserum) dazugegeben, wodurch es zu einer Brückenbildung und somit zur sichtbaren Agglutination kommt, jedoch nur über eine Zentrifugation.

Die Agglutination kann auf verschiedene Weisen sichtbar gemacht werden. Ein bisher bekanntes Verfahren zur Blutgruppenbestimmung verwendet beispielsweise Rundboden-Mikrotiterplatten, wobei in die Vertiefungen der Platte eine Serie von verschiedenen Antiseren, die spezifische Antikörper als spezifische Bindungspartner für die Antigene auf der Erythrozytenmembran enthalten, einpipettiert und mit einer Testflüssigkeit versetzt wird, welche die zu testenden Erythrozyten in verdünnter Form enthält. Nach einer Inkubationszeit wird die Platte anzentrifugiert und anschließend vorsichtig aufgeschüttelt. Bei einer positiven Antigen-Antikörper-Reaktion haben sich die Erythrozyten zu einem Klumpen agglutiniert, der sich durch den Zentrifugierschritt in der Mitte der Vertiefung ansammelt und durch das Aufschütteln möglichst nicht zerstört wird. Eine positive Reaktion ist demnach als punkt- oder knopfförmige Erythrozytenansammlung in der Mitte einer Vertiefung optisch zu detektieren. Demgegenüber besteht bei einer negativen Antigen-Antikörper-Reaktion, d.h. bei nicht erfolgter Agglutination, keine Verbindung zwischen den Erythrozyten. Die nach dem Zentrifugierschritt in der Vertiefungsmitte angesammelten Erythrozyten verteilen sich demnach durch das Aufschütteln über einen Großteil des U-förmigen Vertiefungsbodens. Eine negative Reaktion ist somit durch einen flächigen, über den Boden der Vertiefung ausgedehnten Erythrozytenfleck optisch detektierbar.

Dieses Verfahren ist jedoch nur aufwendig automatisierbar. Zum einen ist es technisch sehr aufwendig, einen Zentrifugier- und ein Aufschüttelungsschritt zu realisieren. Zum anderen hängt die Zuverlässigkeit der Detektion einer positiven Reaktion von der Stärke der Antikörper-Antigen-Bindung ab: Bei schwachen Bindungen kann der agglutinierte Erythrozytenklumpen durch äußere Störungen, insbesondere durch das Aufschütteln, wieder getrennt und somit fälschlich als negative Reaktion diagnostiziert werden. Aus diesem Grunde ist wegen des eventuell fehlenden klaren Sedimentationsbildes auch die automatische Unterscheidung zwischen positiver und negativer Reaktion, z.B. mittels automatischer photometrischer Auswertung, nicht ohne weiteres möglich, sondern muß von einer erfahrenen Bedienperson vorgenommen oder zumindest überprüft werden. Schließlich sind die Reaktionsbilder nicht stabil, da sich die Trägererythrozyten mit und ohne Agglutionation stets an der tiefsten Stelle des Reaktionsgefäßes ansammeln. Somit ist keine zuverlässige Überprüfung des Reaktionsergebnisses möglich. Außerdem ist auf Grund der inkompletten Antikörper keine sichtbare Reaktion und somit Detektion für das Antikörper-Screening gegeben. Ebenso ist die Kreuzprobe, Blutverträglichkeitstest, nicht gegeben.

Ein weiteres Verfahren verwendet zur Detektion von Antikörper-Antigen-Reaktionen speziell präparierte Mikrotiterplatten, bei denen in den V-förmigen Boden der einzelnen Vertiefungen jeweils eine treppenförmige Struktur eingefräst ist. Die Breite der einzelnen Treppen liegt in der Größenordnung von einigen Mikrometern. In den Vertiefungen der Platte werden zur Durchführung einer Blutgruppenbestimmung in bekannter Weise Antiseren bzw. Testerythrozytenlösungen mit Erythrozyten- bzw. Blutserumsproben zur Reaktion gebracht und nach einer Inkubation bei Temperaturen von etwa 30° C ausgewertet. Findet in einer Vertiefung keine Antikörper-Antigen-Reaktion statt, so sammeln sich die (Test-)Erythrozyten, die hier die Träger von Antigenen sind, in der Mitte der V-förmigen Vertiefungen und sind als punkt- oder knopfförmiges Sediment detektierbar. Bei einer positiven Reaktion vernetzen jedoch die Erythrozyten und bilden ein flächig agglutiniertes Gebilde, das durch die treppenförmige Struktur gehalten wird. Dieselbe Reaktion in einer V-Boden-Mikrotiterplatte ohne Treppenstruktur würde zu einem Erythrozytenklumpen in der Vertiefungsmitte führen und wäre somit nicht von einer negativen Reaktion unterscheidbar. In diesem Fall ist eine positive Reaktion aufgrund der treppenförmigen Struktur jedoch als flächiger, über den Boden der Vertiefung ausgedehnter "Teppich" aus agglutinierten Erythrozyten optisch detektierbar.

Dieses Verfahren ist als klassische Agglutinationsmethode, bei welcher keine zusätzlichen mechanischen Eingriffe, wie Zentrifugieren, notwendig sind, relativ gut automatisierbar. Auch ist die Auswertung aufgrund klarer Sedimentationsbilder, die gegenüber mechanischen Störungen relativ stabil sind, automatisiert photometrisch oder mit einer Videokamera möglich. Problematisch ist hingegen der hohe Aufwand zur Herstellung der betreffenden Mikrotiterplatten durch Einfräsen der Treppenstruktur. Aus diesem Grunde liegen die Preise der Mikrotiterplatten so hoch, dass eine einmalige Verwendung wirtschaftlich nicht tragbar ist. Zum Mehrwegbetrieb hingegen sind aufwendige Reinigungs- und Sterilisationsschritte notwendig, um keine verfälschten Ergebnisse zu erhalten. Weiterhin ist die Lebensdauer der Mikrotiterplatten erfahrungsgemäß auf etwa 50 bis 80 Einsätze begrenzt. Problematisch ist des weiteren die Erfassung von schwach-positiven Reaktionen, bei denen man eine Verweigerungswahrscheinlichkeit von etwa 5 % beobachtet. Dies ist besonders bei der Serumgegenprobe zu beobachten, da der individuelle Antikörper (Isoagglutinin) -Titer von Mensch zu Mensch stark variiert. Auch hier ist kein Antikörper-Screening und kein Blutverträglichkeitstest möglich.

Zum Nachweis von Antigenen oder Antikörpern ist aus der US 5.460.940 (EP 0305337B1) ein Verfahren zur optischen Sichtbarmachung von Komplexen von trägergebundenen Antigenen mit Antikörpern in Reaktionsgefäßen bekannt geworden, welche ID-Gel-Technik genannt wird. Diese benützt sechs parallele, mit Gel-Beads, zum Beispiel Agarose, gefüllte transparente Säulen in Form einer Karte. Im oberen Bereich oberhalb der Säule befindet sich ein zur Aufnahme der Probe dienendes, größeres Behältnis, in welchem sich die bekannten, für die nachzuweisenden Antigene bzw. Antikörper spezifischen Bindungspartner befinden, auf die eine vorbestimmte Menge einer Testflüssigkeit trägergebundener Komplexe zugefügt wird, die nachzuweisende Antigene bzw. Antikörper an Träger gebunden enthält. Anschließend wird die Mischung zur Sedimentation einem Zentrifugierschritt ausgesetzt; die Sichtbarmachung einer Reaktion ist nur über einen solchen Zentrifugierschritt möglich. Durch normale Gravitation wird eine Sedimentation zum tiefsten Punkt des Gefäßes hin nicht erreicht.

Zwischen der unteren, mit Gel gefüllten Säule und dem oberen Behältnis muß bei Befüllung mit der Probe ein Luftpolster verbleiben; es erfolgt eine Ruhe-Inkubationsphase von 15 Min bei 37 Grad C; die anschließende Zentrifugationszeit der Karte beträgt 10 Minuten bei 85xg. Das Ergebnis wird durch eine seitliche Betrachtung der Säule abgelesen. Bei positiver Reaktion befinden sich die Agglutinate auf dem Gel der Säule oder im Gel der Säule flockig verteilt. Bei einer negativen Reaktion befinden sich die Erythrozyten in kompakter Form am Boden der Säule. Eine Verkürzung der Inkubationszeit ist nicht möglich, weil die Proben nicht mechanisch bewegt werden dürfen, weil sonst die Probe während der Inkubationsphase direkt mit dem Gel in Kontakt käme und dadurch eine falsch-negative Reaktion angezeigt würde. Auch kann die g-Zahl nicht erhöht und damit die Zentrifugationszeit nicht erniedrigt werden, weil sonst ebenfalls eine falsch-negative Reaktion angezeigt würde. Ebenso sind für die Serumgegenprobe und AB0-RH System gesonderte Karten notwendig, wie für die Unterscheidung von zum Beispiel IgG und IgM gesonderte Säulen einer Karte notwendig sind. Das Gefäß wird zentrifugiert und danach der Ort optisch ermittelt, an dem sich die Träger ansammeln. Sammeln sich diese oberhalb der inerten Partikelschicht an, so weist dies auf eine stark positive Antigen-Antikörper-Reaktion hin: Die zu einem Agglutinat vernetzten Träger werden durch die Partikel daran gehindert, gemäß der Zentrifugalkräfte zum Boden des Gefäßes durchzudringen. Bei negativer Antigen-Antikörper-Reaktion dringen dagegen die nicht vernetzten Träger zum Gefäßboden vor, was bedeutet, dass ohne einen Zentrifugierschritt eine Reaktion nicht erkannt werden kann. Anschließend können die Agglutinationsreaktionen identifiziert werden, indem das Reaktionsgefäß von der Seite betrachtet und die Lage der Träger optisch erfaßt wird.

Bedingt durch die noch immer lange Inkubations- und Zentrifugationszeit und der Kartenform sowie gesonderter Säulen für die einzelnen Bestimmungen ist diese Technik für eine Vollautomation nicht gut geeignet. Auch ist die Versendung der Karten zu den Kunden problematisch, da das Gel bei einem Aufdemkopfstehen der Säulen aus denselben herauslaufen kann. Auch läßt sich das Verfahren nur umständlich automatisieren, da ein Zentrifugierschritt notwendig ist. Problematisch ist des Weiteren die Erfassung der schwachen Antigen-Antikörper-Reaktionen, bei denen eine ungleichmässige, flockige Verteilung von Agglutinat über die Füllhöhe der Partikel beobachtet wird. Diese Reaktionen lassen sich daher nicht zuverlässig automatisch erfassen.

### Technische Aufgabe:

Der Erfindung liegt die Aufgabe zugrunde, unter Vermeidung der Nachteile des Standes der Technik ein Verfahren zum Nachweis von Antikörpern oder Antigenen sowie zur Blutgruppenbestimmung anzugeben, welches einfach durchführbar und leicht automatisierbar ist sowie zu höchst zuverlässigen Testergebnissen führt.

### Offenbarung der Erfindung sowie deren Vorteile:

Die Aufgabe wird bei einem Verfahren der eingangs genannten Gattung dadurch gelöst, dass
1.1 die Reaktion in einem oben offenen und am Boden geschlossenen Mikroreaktionsgefäß durchgeführt wird, das wenigstens in seinem bodennahen Bereich einen sich verjüngenden Querschnitt aufweist, so dass das Gefäß wenigstens in seinem bodennahen Bereich wenigstens teilweise eine schräge Wandung besitzt, die in den Boden übergeht bzw. den Boden bildet,
1.2 mindestens in diesen bodennahen Bereich mit schräger Wandung des Mikroreaktionsgefäßes eine viskose, homogene Substanz, insbesondere erhitzte Gallerte oder erhitztes Gel, eingegossen und erkalten gelassen wird, so dass die Substanz nach dem Erkalten den Bereich des Gefäßes mit schräger Wandung einschließlich des Bodens als dünne, homogene Schicht auf der schrägen Wandung des Gefäßes oder in Form eines Meniskus bedeckt bzw. ausfüllt und dabei eine feine Vernetzung bzw. Gitterstruktur ausbildet;
1.3 ein vorbestimmtes Volumen der Testflüssigkeit in das Gefäß auf die homogene Schicht der viskosen Substanz aufgegeben wird, wobei der spezifische Bindungspartner entweder der Testflüssigkeit oder der viskosen Substanz zugesetzt ist oder eine weitere Flüssigkeit, die den spezifischen Bindungspartner enthält, vor oder nach Zufügen der Testflüssigkeit dem Gefäß zugefügt wird;
2.4 das Mikroreaktionsgefäß wenigstens so lange inkubiert wird, bis sich in der Grenzschicht zwischen der schrägen Wandung des Mikroreaktionsgefäßes und der dünnen, homogenen Schicht aus der viskosen Substanz allein aufgrund der Schwerkraft das sichtbare Sediment/Agglutinat ausbildet, wobei eine insbesondere auf der schrägen Wandung des Gefäßes sich flächig ausbildende Agglutinatschicht auf eine positive Antigen-Antikörper-Reaktion hinweist und ein insbesondere im Bereich des tiefsten Punktes des Bodens bzw. der schrägen Wandung des Gefäßes sich bildende, räumlich gerinfügig ausgedehnte Ablagerung auf eine negative Reaktion ohne Antigen/Antikörper-Komplex hinweist.

In weiterer erfindungsgemäßer Ausgestaltung wird als viskose Substanz ein Gel oder eine Gallerte verwendet, das bzw. die bis zur Schmelztemperatur erhitzt und in flüssigem Zustand zur Ausbildung der homogenen Schicht in das Mikroreaktionsgefäß eingefüllt wird, wo das Gel bzw. die Gallerte abkühlt und dadurch eine homogene Schicht gebildet wird.

Es wird somit ein Mikroreaktionsgefäß mit einem sich von oben nach unten verjüngenden Querschnitt verwendet, welches die viskose Substanz enthält; ein vorbestimmtes Volumen der Testflüssigkeit wird auf die viskose Substanz dem Gefäß zugefügt, wobei der spezifische Bindungspartner entweder der Testflüssigkeit oder der viskosen Substanz zugesetzt wird, oder eine weitere Flüssigkeit, die den spezifischen Bindungspartner enthält, vor oder nach Zufügen der Testflüssigkeit dem Gefäß zugefügt wird; das sich ausbildende oder ausgebildete Sediment/Agglutinat in Aufsicht von oben oder unten auf das Mikroreaktionsgefäß ausgewertet wird; wobei ein flächiges Agglutinat (Rasenbildung) von Antigenen bzw. Antikörpern, Bindungspartnern und Trägern auf eine positive Antigen-Antikörper-Reaktion hinweist und eine räumlich geringfügig ausgedehnte Ablagerung (Knopfbildung) von Trägern auf eine negative Reaktion mit Abwesenheit eines Antigen/Antikörper-Komplexes hinweist.

Die viskose Substanz kann ein Gel sein, das bis zur Schmelztemperatur erhitzt und in flüssigem Zustand zur Ausbildung der homogenen Schicht in das Mikroreaktionsgefäß eingefüllt wird, wo die viskose Substanz bis zum Gelpunkt bzw. in die Nähe des Gelpunktes abkühlt und dadurch eine homogene Schicht gebildet wird, die beim Abkühlen eine feine Vernetzung bzw. Gitterstruktur ausbildet.

Die viskose Substanz ist vorzugsweise ein Gel, bevorzugt ein organisches Gel, insbesondere Agarose und/oder Gelatine. Die viskose Substanz kann auch aus vernetzten Polymeren bestehen, wobei jede viskose Substanz, welche aus vernetzten Polymeren besteht, geeignet ist.

Das Gel - vorzugsweise vom Typ XII low viscosity for beading NRA 7299 - weist wenigstens eine Gelstärke von 0,01 g/cm² = ca. 0,02 mg/100 ml Gelkonzentration auf. Die bevorzugte Konzentration liegt zwischen 0,1 mg/100 ml und 6666mg/100 ml Gelkonzentration. Insbesondere wird der Bereich bevorzugt: 10 mg/100 ml bis 110 mg/100 ml Gelkonzentration. Darin liegt ein besonders bevorzugter Bereich zwischen: 40 mg/100 ml bis 80 mg/100 ml Gelkonzentration.

Wenn das erfindungsgemäße Verfahren zur Durchführung einer Serumgegenprobe, die Isoagglutinine beinhaltet, oder zum Nachweis von irregulären Blutgruppenantikörpern (Antikörper-Suchtest) oder zur erythrozytenseitigen Blutgruppenbestimmung mittels bekannter Antikörper dient, dann enthält die Testflüssigkeit Blutserum mit unbekannten Antikörpern oder unbekannte Erythrozyten. Der Testflüssigkeit oder der viskosen Substanz wird eine Flüssigkeit zugefügt, die Testerythrozyten oder Testantikörper einer bekannten Blutgruppe enthält. Im speziellen Fall, zum Beispiel für die Serumgegenprobe, sind die Träger mit spezifischen Bindungspartner-Antigenen bekannter Blutgruppe besetzt, die den nachzuweisenden Antikörper (Isoagglutinin) nachweisen. Bei Blutgruppenantikörpern (Antikörper-Suchtest) sind es ebenfalls spezifische Bindungspartner-Antigene, die auf den Erythrozyten sitzen. Diese zeigen dann den irreguären Antikörper an. Bei erythrozytenseitiger Blutgruppenbestimmung ist das Trägerantigen nicht bekannt, sondern in der Testflüssigkeit oder in der viskosen Substanz befindet sich der bekannte Suchantikörper.

Einer der Flüssigkeiten bzw. dem Gemisch oder der viskosen Substanz können zum Zeitpunkt der Untersuchung Proteine, wie Protein A und/oder G, und/oder Antigene, und/oder Antikörper (Anti-IgG (Coombsserum), Anti-C3d, Anti-IgM) zugesetzt werden, was Einfluß auf die Reaktion nimmt. Die Träger innerhalb der Testflüssigkeit sind Erythrozyten, Leukozyten, Blutplättchen, Latex-Partikel oder Agarose-Partikel, Glaspartikel oder Kunststoffpartikel. Wenn die benötigten Inhaltsstoffe in der Flüssigkeit enthalten sind, so wird nur Nativgel benötigt.

In vorteilhafter Weise kann somit das erfindungsgemäße Verfahren zur erythrozytenseitigen Blutgruppenbestimmung, zur Bestimmung von irregulären Antikörpern und zur Serumgegenprobe eingesetzt werden. Hier besteht grundsätzlich das Problem, dass jeder Spender einen individuellen Antikörper-Titer hat. Um mit herkömmlichen Verfahren eine zuverlässige Reaktion zu erhalten, müßte für jeden Spender eine individuelle Verdünnung des Spenderserums erstellt werden, was insbesondere für die Automatisierung des Verfahrens hinderlich ist.

Die Querschnittsfläche des Gefäßes beträgt im Bereich der Füllhöhe der viskosen Substanz wenigstens das Vierfache der Querschnittsfläche des Gefäßes im Bereich unterhalb der viskosen Substanz. Es werden Spitzboden- oder Rundboden-Mikrotiterplatten verwendet, wobei das Mikroreaktionsgefäß eine Vertiefung einer Spitzboden- oder Rundboden-Mikrotiterplatte ist.

Verfahrenmäßig wird nach dem Schritt 1.2. inkubiert, vorzugsweise bei Temperaturen von wenigstens 5 Grad C, bevorzugt zwischen 10 und 37 Grad C, insbesondere bei Raumtemperatur, und für wenigstens 15 Minuten, bevorzugt wenigstens 50 Minuten.

Eine der Flüssigkeiten oder die viskose Substanz kann verfahrensgemäß Albumin in einer Konzentration von wenigstens 0,01 mg/ml, vorzugsweise zwischen 0,2 mg/ml und 1 mg/ml, enthalten.

Das erfindungsgemäße Verfahren hat den großen Vorteil, dass es sich als nur der Schwerkraft unterliegendes Sedimentationsverfahren leicht automatisieren läßt. Das basiert auf der Tatsache, dass das über den Schmelzpunkt (d.h. mindestens bis zur Melting-Temperatur) erhitzte und in das Mikroreaktionsgefäß gegossene Gel oder die Gallerte beim Erkalten eine irreguläre Mikrovernetzung bzw. Mikro-Gitterstruktur ausbildet, die unter dem Elektronenmikroskop zu sehen ist und die quasi als Sieb oder Bremse für die nachzuweisenden Antikörper oder Antigene in der Testflüssigkeit, für die Bindungspartner (Nachweis-Antigene bzw. Nachweis-Antikörper) und/oder für die Träger (beispielsweise Erytrozyten, Leukozyten, Blutplättchen, Latex-Partikel oder Agarose-Partikel) fungiert, d.h. durch die die Reaktionspartner nur gebremst hindurchdiffundieren können. Dabei kommt es im Fall einer positiven Antikörper-Antigen-Reaktion mit Agglutination der Träger zur Ausbildung einer Agglutinationsschicht, Agglutinatrasen oder Agglutinatteppich, in der Grenzschicht zwischen Gefäßwand und Schicht aus viskoser Substanz, deren Ausdehnung in Länge und Breite wesentlich begünstigt ist gegenüber der Ausdehnung in die Höhe, d.h. gegenüber einer Verdickung.

Mit anderen Worten: Einer der entscheidenden Kernpunkte der Erfindung besteht in der Erkenntnis des Erfinders, dass die viskose Substanz als homogene, vorzugsweise dünne, Schicht oder in Form eines Meniskus auf der schrägen Wandung des Mikroreaktionsgefäßes aufzubringen ist. Denn dann bildet eine Reihe von viskosen Substanzen, wie z.B Gel oder Gallerte, beim Abkühlen auf den Festpunkt (Gelpunkt) eine Art feinster, fibröser netz- oder gitterartiger Strukturen in unregelmäßiger Weise aus, in denen sich Flüssigkeit befindet. Die in dem Testgemisch bzw. in der Testflüssigkeit enthaltenen Antigene oder Antikörper bilden mit den ebenfalls in dem Testgemisch bzw. in der Testflüssigkeit oder innerhalb der viskosen Schicht befindlichen spezifischen Bindungspartnern Komplexe, welche allein aufgrund der Schwerkraft die homogene Schicht aus viskoser Substanz durchwandern und dabei innerhalb der Schicht mit den spezifischen Bindungspartnern (den Nachweis-Antigenen bzw. den Nachweis-Antikörpern), die sich entweder ebenfalls innerhalb des Testgemisches bzw. der Testflüssigkeit oder innerhalb der viskosen Substanz oder innerhalb einer weiteren Flüssigkeit befinden, reagieren. Diese viskose Substanz wirkt auf die aufgrund der Schwerkraft absinkenden komplexen Reaktionspartner wie eine verzögernde Bremse. In der Grenzschicht auf der schrägen Wandung des Gefäßes schlägt sich im Fall einer positiven Reaktion ein Agglutinat (Sedimentation) in Form einer zusammenhängenden, flächigen Schicht nieder. Ein auf der schrägen Wandung sich flächig ausbildendes Agglutinat - Rasenbildung genannt - von nachzuweisenden Antigenen bzw. Antikörpern, spezifischen Bindungspartnern (Nachweis-Antikörper bzw. Nachweis-Antigene) und Trägern (beispielsweise Erythrozyten) weist auf eine positive Antigen-Antikörper-Reaktion hin. Bei einer negativen Reaktion ergibt sich kein Agglutinat, sondern es bildet sich eine räumlich weit weniger ausgedehnte Ablagerung (von Trägern) aus - Knopfbildung genannt -, die sich in der Regel im Bereich des tiefsten Punktes der schrägen Wandung im Boden ausbildet; das weist auf eine negative Reaktion hin. Deshalb genügt es gemäß der Erfindung, dass die viskose Substanz in Form einer homogenen Schicht, zum Beispiel einer Gelschicht, sich nur im Bereich dieser schrägen Wandung befindet, wobei allerdings diese viskose Schicht auch dicker, im Bereich einiger Millimeter, gewählt sein kann oder auch einen Meniskus ausbilden kann.

Die Schicht aus der viskosen Substanz hat also die Aufgabe, eine infolge Antigen-Antikörper-Reaktion erfolgte Agglutination der Antigen- und/oder Antikörper-Träger, d.h. die vernetzten Antigen- und/oder Antikörper-Träger, in Form einer flächigen Schicht ("Teppich" bzw. "Rasen") im Bereich der V-Vertiefung oder bombierten Vertiefung sichtbar zu machen und zu halten. Dagegen wandern unvernetzte Träger gemäß der bloßen Schwerkraft an der schrägen Wandung entlang und sammeln sich vorzugsweise (weil das Gefäß sich verjüngt) an dem tiefsten Teil des Gefäßbodens unter Ausbildung eines räumlich erheblich weniger ausgedehnten, im Idealfall punktförmigen, optisch erfaßbaren Gebildes ("Knopf") an.

Auch schwächere Reaktionen können mit dem erfindungsgemäßen Verfahren nachgewiesen werden. Hierfür ist die viskose Substanz mit einer entsprechend erhöhten Viskosität auszubilden und eine entsprechend lange Sedimentations-/Inkubationszeiten bis zum Sichtbarwerden der Reaktion zu veranschlagen.

Wenn eine Rasenschicht innerhalb der Grenzschicht ausgebildet ist (was einer positiven Reaktion (Agglutinat) entspräche), aber die negative Reaktion noch nicht als Knopf vorliegt, sondern mehr oder weniger am Boden als flächige Verteilung vorliegt, so kann diese Reaktion in dieser Grenzschicht durch erhöhte Schwerkraft, zum Beispiel durch Zentrifugation, erzwungen werden, so dass sich die nichtreagierten Erythrozyten schneller in der tiefsten Schicht anzusammeln.

In das Gel können erfindungsgemäß verschiedenste Puffer und/oder auch die spezifischen Bindungspartner (Proteine, wie Protein A und/oder G, und/oder Antigene und/oder Antikörper, Nachweis-Antigene oder - Antikörper, insbesondere monoklonale Antikörper, und/oder die Träger) eingebaut werden, welche für die Antikörper- bzw. Antigen-Reaktion förderlich sind. Dabei können sowohl Puffer als auch Bindungspartner schon im Erhitzungsstadium des Gels zugefügt werden, vorausgesetzt dass sie hitzestabil sind.

Des weiteren besitzt das Verfahren den Vorteil, dass als Mikroreaktionsgefäße herkömmliche Mikrotiter-Wegwerfplatten mit V-förmiger oder bombierter Vertiefung zum Einsatz kommen können, die kostengünstig und mit einem Pippettierroboter leicht zu handhaben sind. Diese müssen zur Durchführung des Verfahrens lediglich mit der viskosen Substanz, beispielsweise dem Gel oder der Gallerte, unter Ausbildung einer homogenen Schicht auf der schrägen Wandung innerhalb der V- oder bombierten Vertiefung der Mikrotiter-Wegwerfplatten präpariert werden.

Die Auswertung erfolgt entweder mit dem bloßen Auge oder insbesondere vollautomatisch mit einer Video- oder CCD-Kamera oder photometrisch durch Aufsicht bzw. Unteransicht auf das Gefäß. Die photometrische Auswertung ist unter geringerem technischen und wirtschaftlichen Aufwand durchführbar als die Aufzeichnung von Sedimentationsbildern mit einer Kamera und deren Auswertung und eignet sich somit für eine einfachere und kostengünstigere Automatisierung des Verfahrens.

Die viskose Substanz kann ein Gel sein, welches auf oder über den Schmelzpunkt erhitzt und anschließend in die Gefäße zu Bildung einer homogenen Schicht eingefüllt wird. Der viskosen Substanz können Stoffe beigefügt sein, welche auf die Agglutinationsreaktion Einfluß nehmen, so z.B. auch der spezifische Bindungspartner des nachzuweisenden Antikörpers oder nachzuweisenden Antigens, d.h. das spezifische Nachweis-Antigen oder der spezifische Nachweis-Antikörper.

Die Viskosität der viskosen Substanz ist so zu wählen, dass eine Antigen-Antikörper-Reaktion zu einer sichtbaren, flächigen Agglutination der Träger in der Grenzschicht führt. Dies kann insbesondere von der Stärke der Antigen-Antikörper-Bindung und von der abstoßenden Wirkung der Träger abhängen. Bei schwacher Antigen-Antikörper-Bindung ist in der Regel eine höhere Viskosität erforderlich als bei einer starken Bindung. Das Verfahren hat jedoch den Vorteil, dass es prinzipiell für sämtliche Reaktionsstärken geeignet ist.

Insgesamt müssen die Reaktionsbedingungen, d.h. Viskosität der viskosen Substanz, Konzentration der Antigene bzw. Antikörper und der spezifischen Bindungspartner, Umgebungstemperatur und dergleichen, so gewählt sein, dass bei einer positiven Antigen-Antikörper-Reaktion eine Agglutination der Träger in der Grenzschicht vorliegt. Die jeweiligen Bereiche hängen insbesondere von der zu untersuchenden Reaktion ab. Dazu kann des weiteren vorteilhaft sein, die Probe nach dem Zusammenfügen der Reagenzien bei geeigneter Temperatur für einige Zeit zu inkubieren, damit sich das Sedimentationsbild ausbildet.

Der Zusatz von Coombsserum, welches Anti-IgG enthält, was zur sichtbaren Agglutination führt, ist besonders hilfreich bei inkompletten Antikörpern, die ansonsten keine Reaktion zeigen würden. Coombsserum setzt allerdings die Abstoßungskräfte nicht herab.

Die Flüssigkeit bzw. die Flüssigkeiten sollten blasenfrei auf die Schicht der viskosen Substanz zugefügt werden, was beim vollautomatisch durchgeführten Verfahren durch eine Pippettiervorrichtung mit geeigneter mechanischer Steuerung, z.B. einen Pippettierroboter, erreicht werden kann.

In einer vorteilhaften Weiterbildung der Erfindung ist das Gel ein organisches Gel, insbesondere Agarose und/oder Gelatine. Diese Gele haben den Vorteil, dass sie sehr leicht anzusetzen sind, insbesondere in Verbindung mit Wegwerf-Mikrotiterplatten. Sie lassen sich des weiteren gut lagern, sind stabil und kurzfristig relativ unempfindlich gegenüber Änderungen der Umgebungstemperatur. Insbesondere mit Agarose einer Gelkonzentration von etwa 40 mg/100 ml bis 80 mg/ml wurden bei der Durchführung von Serumgegenproben und Ak-Suchtesten zur Blutgruppenbestimmung ausgezeichnete Ergebnisse erzielt. Derart mit Agarose präparierte Mikrotiterplatten lassen sich gekühlt einige Monate lagern. Auch die fertig präparierten Proben lassen sich gekühlt einige Wochen lagern, wobei die Reaktionsbilder stabil bleiben. Die Reaktionsergebnisse können somit auch längere Zeit nach Durchführung des Tests zur Kontrolle herangezogen werden.

Die Träger, an welche die nachzuweisenden Antigene bzw. Antikörper oder die spezifischen Bindungspartner gebunden sind, sind vorzugsweise Erythrozyten, Leukozyten, Blutplättchen oder Latex-Partikel. Die Latex-Partikel können zur einfacheren optischen Auswertung sichtbar gefärbt oder fluoreszenz-markiert sein. Die Konzentration der Träger in der Reaktionsflüssigkeit ist so zu wählen, dass das Agglutinat mit der gewählten optischen Detektionsmethode zuverlässig detektierbar ist.

Erfindungsgemäß wird das Sedimentationsbild optisch durch Aufsicht von oben oder unten auf das Reaktionsgefäß ausgewertet. Falls von unten gemessen wird, muß das Mikroreaktionsgefäß transparent sein. Es ist vorzugsweise auch transparent, falls von oben ausgewertet wird, um den Kontrast der Träger zum Hintergrund zu erhöhen. Durch die unterschiedlichen Gefäßquerschnitte im oberen Bereich der viskosen Substanz und in deren unterem Bereich bzw. im Bereich des Gefäßbodens wird im Falle einer Agglutination in der Grenzschicht ein flächiger "Teppich" bzw. "Rasen" aus vernetzen Trägern erzeugt, während sich die Träger in einem in der Aufsicht kleineren Bereich ansammeln, wenn keine Agglutination erfolgt. Die Antigen-Antikörper-Reaktion kann somit optisch sehr einfach detektiert werden durch Messung der räumlichen Ausdehnung der Ansammlung der Träger. Das Sedimentationsbild kann visuell durch eine Bedienperson oder automatisch auf photometrischem, flurometrischem Weg oder videogesteuert analysiert werden. Da die Träger-Ansammlung im wesentlichen von oben oder unten vermessen wird, ist eine besonders gute Automatisierbarkeit des Verfahrens gegeben. Insbesondere Mikrotiterplatten mit einer Mehrzahl von Mikroreaktionsgefäßen, die in einer Ebene angeordnet sind, lassen sich in Aufsicht von oben oder unten auf die Platte besonders gut in einem Arbeitsgang auswerten.

Die Deutlichkeit des sichtbaren bzw. detektierbaren Reaktionsergebnisses hängt unter anderem von der gewählten Gefäßform ab. Daher ist das Gefäß, zumindest im Bereich der Schicht aus viskoser Substanz, sich konisch verjüngend ausgebildet und besitzt eine schräg verlaufende oder schräg umlaufende bzw. konische Wandung, z.B. eine V-Vertiefung einer Spitzboden-Mikrotiterplatte oder eine bombierte, gerundete Vertiefung einer Rundboden-Mikrotiterplatte, die ja in ihrem unteren Bereich eine schräge Wandung aufweist, wobei die Schräge im Schnitt eine schiefe Ebene oder auch eine gekrümmte Kurve sein kann. Gute Ergebnisse werden allgemein erzielt, wenn die Querschnittsfläche des Gefäßes im Bereich der Füllhöhe der viskosen Substanz wenigstens das Vierfache der Querschnittsfläche des Gefäßes im Bereich unterhalb der viskosen Substanz beträgt.

Vorzugsweise erfolgt die Analyse, ob in einem Mikroreaktionsgefäß eine Antikörper-Antigen-Reaktion stattgefunden hat, photometrisch, indem die Absorption des in der Vertiefung enthaltenen Gemischs entlang einer vorbestimmten Linie gemessen und aufgezeichnet und das so ermittelte örtliche Absorptionsprofil ausgewertet wird. Diese Auswertemethode ist gut für die Auswertung einer Vielzahl von Proben, z.B. einer Mikrotiterplatte, in einem Arbeitsgang und damit in einer automatisierten Form des Verfahrens geeignet. Indem nur ein Absorptionsprofil gemessen wird, ist die zu speichernde und auszuwertende Datenmenge gegenüber beispielweise Videoauswertung verringert. Vorzugsweise wird dabei für eine feste Profilbreite die Profillänge ermittelt. Eine positive bzw. negative Reaktion wird dann diagnostiziert, wenn die Profillänge unterhalb bzw. oberhalb eines vorbestimmten Wertes liegt. Diese Auswertemethode hat sich als besonders zuverlässig erwiesen.

Das Mikroreaktionsgefäß kann auch - insbesondere im Bereich seiner schrägen Wandung - mit einer zusätzlichen Kodierung versehen sein, so dass in der Grenzschicht eine Kodierung angeordnet ist. Diese Kodierung kann insbesondere aus Antiglobulinen - gegen Immunglobuline gerichtete Antikörper -, sogenannten "Fang-Antikörpern" wie zum Beispiel mit Anti-IgG oder Protein A und/oder G mit hoher Bindungsaffinität für Immunglobuline oder Anti-IgM, Komplement, Anti-C3d und so weiter, bestehen bzw. diese enthalten. Eine solche zusätzliche Kodierung ist besonders dann von Vorteil, wenn Reaktionen nachgewiesen werden sollen, bei denen die Antikörper spezifisch klein sind, wie das zum Beispiel bei inkompletten IgG-Antikörpern der Fall ist, oder bei schwachen Antikörpern. Bei starken Antikörpern vom IgG-Typ sowie bei kompletten Antikörpern, wie zum Beispiel beim IgM-Typ, benötigt man keine Kodierung.

Eine derartige zusätzliche Kodierung ist außerdem dann von Vorteil, wenn freie Antikörper weggefangen werden sollen. Ebenso sind Kombinationen denkbar von zusätzlicher Kodierung der schrägen Wandung und von spezifischen Inhaltsstoffen in der viskosen Substanz; im Prinzip ist eine praktisch beliebige Kodierung von Gefäßwand und Zugabe in die viskose Substanz möglich.

Das Bild der Reaktionsgefäße kann alternativ auch mittels einer Video- oder CCD-Kamera oder dergleichen aufgezeichnet werden. Durch Bildanalyse können positive von negativen Reaktionen unterschieden werden. Eine weitere Möglichkeit zur Erfassung der Reaktionsbilder ist die Fluoreszenzmarkierung der Träger und Detektion der räumlichen Emission von Fluoreszenzlicht.

Vorzugsweise enthält eine der Flüssigkeiten oder die viskose Substanz Albumin in einer Konzentration von wenigstens 0,01 mg/ml, vorzugsweise zwischen 0,2 mg/ml und 1 mg/ml, oder ein anderes Supplement. Dieses dient zur Stabilisierung der Erythrozyten und zur Reduzierung der Abstoßungskräfte der Erythrozyten untereinander, wodurch eine bessere Vernetzung erreicht wird. Des weiteren wird durch Albumin-Zusatz die Diffusionsgeschwindigkeit der Erythrozyten durch das viskose Medium verringert und damit die Empfindlichkeit erhöht.

Ebenso kann Liss oder Bromealin oder Polyethilenglykol oder ähnliche Substanzen zugesetzt werden, die ähnliche Eigenschaften zur Förderung der Antigen-Antikörper-Reaktion haben. Bei der Blutgruppenbestimmung wird vorzugsweise etwa bei Raumtemperatur inkubiert. Es entfallen somit Inkubatoren, die eine konstante erhöhte Umgebungstemperatur herstellen. Die Automatisierung des Verfahrens wird somit weiter erleichtert.

Ein erfindungsgemäßes Mikroreaktionsgefäß mit einer Vielzahl von Vertiefungen, zum Nachweis von Antikörpern und/oder Antigenen in einer Testflüssigkeit sowie zur Blutgruppenbestimmung durch Reaktion mit einem vorgegebenen spezifischen Bindungspartner, wobei das Antigen bzw. der Antikörper oder der spezifische Bindungspartner in der Testflüssigkeit ungebunden oder an einen Träger gebunden ist und wobei bei positiver Antigen-Antikörper-Reaktion ein Agglutinat aus nachzuweisenden Antigenen bzw. Antikörpern, den entsprechenden spezifischen Bindungspartnern und den Trägern gebildet wird, welches optisch als Sedimentationsbild detektierbar ist, ist dadurch gekennzeichnet, daß sich im Mikroreaktionsgefäß eine viskose Substanz, vorzugsweise Gel oder Gallerte, befindet, welche darin eine homogene Schicht bildet, welche eine feine Vernetzung bzw. Gitterstruktur ausbildet zum Aufbringen eines vorbestimmten Volumes der Testflüssigkeit auf die viskose Substanz.

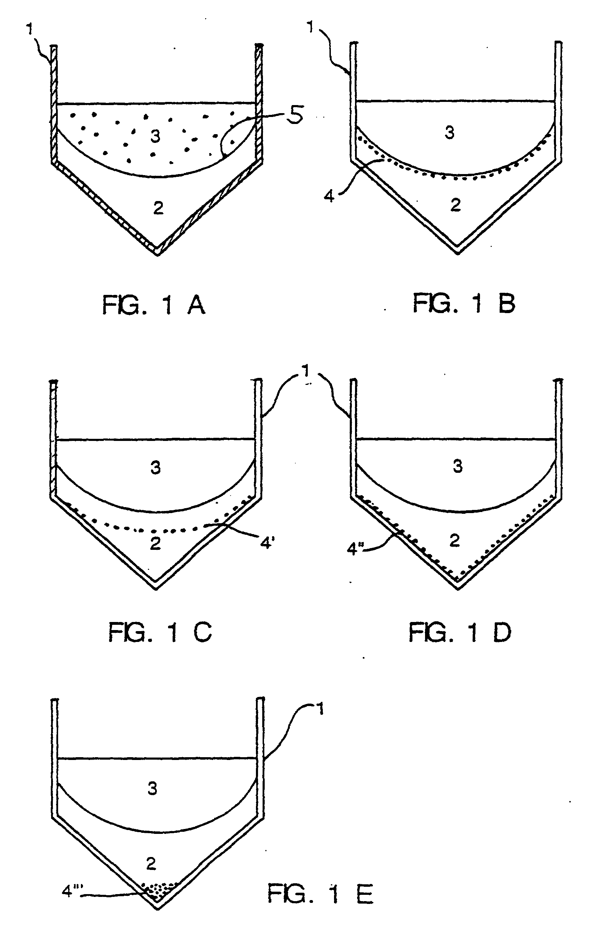

Die Zeichnung zeigt in den Figuren 1A bis 1E schematisch dargestellte Reaktionsgefäße im Längsschnitt, die Vertiefungen in Spitzboden-Mikrotiterplatten sind.

Figur 1 zeigt schematisch Reaktionsgefäße 1 im Längsschnitt, die z.B. Vertiefungen in Spitzboden-Mikrotiterplatten sind. Die Reaktionsgefäße 1 sind im oberen Bereich zylinderförmig und verjüngen sich im unteren Bereich konisch. Sie enthalten bis zu einer bestimmten Füllhöhe eine viskose Substanz 2, insbesondere ein Gel. Bedingt durch den Eingießvorgang, z.B. Schwenken des Gefäßes nach dem Einfüllen des flüssigen Gels und Adhäsion an der Gefäßwandung, ist die Oberfläche der Substanz 2 gekrümmt und bildet einen Meniskus 5 aus. Die Gelschicht sollte wenigstens so dick sein, dass die Wandung des Gefäßes im sich verjüngenden Bereich bedeckt ist. Andererseits bestimmt die Dicke der Gelschicht die Zeit bis Sichtbarmachung einer Reaktion mit. Daher ist eine gekrümmte Gelschicht unter Umständen vorteilhaft, die an allen Stellen möglichst dünn ist. Die dargestellten Größenverhältnisse sind nur schematisch.

Auf die viskose Substanz wird im Verfahrensschritt 1.2 ein bestimmtes Volumen der Testflüssigkeit 3 aufgebracht, welche Träger, Antigene und Antikörper enthält. Die Träger, die zum Nachweis einer Antigen-Antikörper-Reaktion optisch detektiert werden, sind hier stark übertrieben als Punkte dargestellt.
Figur 1A zeigt die Situation kurz nach Zufügen der Testflüssigkeit 3. In der Figur 1 B beginnen die Träger 4 in den oberen Bereichen der Gelschicht 2 zu sedimentieren. In der Figur 1 C sind die Träger 4' bereits in den mittleren Bereich in die Gelschicht 2 abgesunken. Figur 1 D zeigt zweierlei: zum einen eine positive Reaktion und zum anderen haben sich die Träger 4" auf dem V-förmigen Boden zu einem flächigen Agglutinat ausgebildet und sind somit stabil.

Bei negativer Reaktion, also nichtagglutinierend, sind die Träger 4''' auf dem V-förmigen Boden angelangt und wandern (sedimentieren) auf dem Boden in der Grenzschicht zur tiefsten Stelle des Mikroreaktionsgefäßes. In Figur 1 E ist eine negative Reaktion der Träger 4''' dargestellt, deren Ausbildung ggfs. durch Zentrifugieren unterstützt werden kann. Der Schwerkraft/Zentrifugalkraft folgend, sammeln sich die nicht agglutinierten Träger am tiefsten Punkt des Gefässes zu einem räumlich eng umgrenzten Gebilde 4''' an, dass optisch leicht zu identifizieren und von den flächigen Agglutinaten gemäß Figur 1 D zu unterscheiden ist. Selbstverständlich sind die Darstellungen nur schematisch, da der Sedimentierungsprozess kontinutierlich ineinander verschachtelt abläuft.

Im folgenden werden Ausführungsbeispiele beschrieben:
Zur Vorbereitung der Durchführung des Verfahrens werden Mikroreaktionsgefäße mit der viskosen Substanz, die hier Agarose ist, präpariert. d.h. beschichtet. Hierfür wird vorzugsweise folgendermaßen verfahren: 53 mg Agarose, z.B. Agarose Nr. A 7299 Typ XII Firma Sigma, werden auf 100 ml isotonische phosphatgepufferte Kochsalzlösung (z.B. "Suwasol, Fa. Ortho, pH-Wert ca. 7,45 oder PBS, pH 7,4) bei einer Temperatur von etwa 95 Grad C unter Rühren vollständig aufgelöst. Unter diesen Bedingungen weist das entstehende Gel eine Gelkonzentration von etwa 53 mg/100 ml auf. Bei Temperaturen von 50 bis 60 Grad C wird in jede Kavität einer Spitzboden-Mikrotiterplatte etwa 20 µl der Gel-Lösung einpippettiert. Die Platte wird sodann gleichmäßig geschüttelt und im Kühlschrank bei Temperaturen von etwa 4 Grad C bis 8 Grad C ausgelieren gelassen. Das Gel bildet eine Schicht, welche die Gefäßinnenwandung bedeckt. Abgedeckt und gekühlt ist die so präparierte Platte wenigstens etwa zwei bis drei Monate haltbar.

Um die gewünschte Gelkonzentration zu variieren, kann 1 mg bis 2,5 g der genannten Agarose auf 100 ml Flüssigkeit aufgelöst werden. In der Praxis gut bewährt hat sich insbesondere auch ein Gel, das aus 61,5 mg Agarose pro 100 ml PBS (Phosphat Buffered Saline) pH 7,4 hergestellt wird.

Zur Durchführung eines Verfahrens zur Blutgruppenbestimmung, hier der Serumgegenprobe, wird zunächst in vier Mischgefäße jeweils eine bestimmte Menge, z.B. 25 µl, von mit isotonischer Kochsalzlösung im Verhältnis von 1:3,3 verdünntem EDTA-Plasma, Citratplasma oder Serum eines Spenders einpippettiert. Das Verdünnungsverhältnis ist an die photometrische Auswertung aufgrund der Eigenfärbung des Serums angepaßt; bei visueller oder Videoauswertung kann eine kleinere Verdünnung bis hin zum unverdünnten Serum bzw. Plasma verwendet werden. Eine bestimmte Menge, z.B. 25 µl, 1,0 %ige bis 4 %ige Volumenprozent Testerythrozyten der Gruppe A1, A2, B und 0, die hier die Träger der spezifischen Bindungspartner - Antigene A1, A2, B und 0 - sind, werden auf die vier Mischgefäße verteilt und und gemischt. Anschließend wird diese Testlösung in die Mikroreaktionsgefäße, hier die Kavitäten der Mikrotiterplatte, einpippettiert. Das Verdünnungsmedium der Testerythrozyten kann vorzugsweise Rinderalbumin in einer Konzentration von etwa 2 mg bis 150 mg enthalten, hier 100 mg auf 100 ml physiologische Kochsalzlösung NaCl. Alternativ kann das Patientenserum unverdünnt der verdünnten Test-Erythrozytenlösung zugesetzt werden, wodurch ein Arbeitsschritt entfällt. Nach etwa einer Stunde Inkubationszeit bei Raumtemperatur kann das Ergebnis photometrisch ausgewertet werden, indem die Absorption von Strahlung im Rot- bzw. Infrarotbereich als Funktion des Profilortes gemessen wird.

Unter diesen Reaktionsbedingungen wurde im vollautmatischen Verfahren eine Verweigerungsrate < 1 % bestimmt. Bei visueller Auswertung gelang eine noch bessere Identifizierung. Da handelsübliche Mikrotiterplatten in der Regel 96 Kavitäten aufweisen, läßt sich das Verfahren mit hohen Durchsatzwerten automatisieren.

Basierend auf dieser Grundlage kann ein Coombstest (Antikörper-Suchtest /Antiglobulin-Test) ohne Waschen aufgebaut werden, indem im Überschuß Anti-C3d und Anti-IgG zu dem Agarosegemisch (für die Herstellung der Gelschicht auf der Gefäßwand) zugesetzt wird. Das Gemisch aus Suchzelle (spezifisches Antigen und Träger-Erythrozyt) und Spenderserum wird auf das Gel aufpippettiert. Die irregulären Antikörper setzen sich an die Suchzelle, wobei eine Inkubation bei etwa 37 Grad C erfolgt. Bei der folgenden Sedimentation holen sich die beladenen Suchzellen Anti-IgG und sedimentieren durch das Gel bzw. die viskose Schicht gebremst auf die schräge Wandung der Plastikschicht, wo sie dann - und somit in der Grenzschicht - einen Rasen ausbilden.

Das Verfahren kann auch zum Nachweis von IgG-Molekülen verwendet werden, indem Protein A - ein Oberflächenprotein der Bakterienzellwand vieler Stämme von Staphylococcus aureus - oder Protein G - ein Protein der Bakterienzellwand von Streptokokken - zugegeben wird. Protein A und G ist gegenüber hohen Temperaturen sehr stabil und bindet viele Immunglobuline; Protein G hat eine hohe Bindungsaffinität speziell zu Immunglobulinen der Klasse G (IgG). Zur Erfassung von irregulären IgM-Antikörpern kann Anti-IgM zugesetzt werden.

Beim erfindungsgemäßen Verfahren findet die Reaktion in der Grenzschicht zwischen Plastikwand, ggf. auch Plastikboden, des Mikroreaktionsgefäßes und der viskosen Substanz statt, wobei zwei Prinzipien ausführbar sind. Beim ersten Prinzip wird das Verfahren ohne Waschen - und insbesondere ohne Zentrifugieren - durchgeführt. Das Ergebnis ist nach ungefähr einer Stunde ablesbar. Beim zweiten Prinzip kann ein Zentrifugierschritt - wiederum ohne Waschen - zugefügt werden, der die Sichtbarmachung der Ausbildung der negativen Reaktion beschleunigt. Eine Rasenbildung bedeutet dabei jeweils eine positive, eine Knopfbildung eine negative Reaktion.

In beiden Fällen kann eine zusätzliche Kodierung der schrägen Wandung des Mikroreaktionsgefäßes erfolgen, die vor der Beschichtung des Gefäßes mit der viskosen Substanz durchzuführen ist. Die für die Kodierung verwendeten Substanzen werden dabei in fester Weise (kovalent oder in physikalischer Bindung) auf die Wandung aufgebracht, erst anschließend wird die viskose Substanz in das Gefäß gefüllt. Zum Zweck der Durchführung eines Coombs-Tests kommen insbesondere Kodierungen mit Protein A oder G oder einer Mischung aus Protein A und G, zum Beispiel mit einem Mischungsverhältnis 1:1, oder mit Antiglobulinen, gegen Immunglobuline gerichtete Antikörper, wie z.B. Anti-IgG in Betracht, wobei die verwendeten Mikrotiterplatten eine hohe Bindungskraft zu Proteinen aufweisen.

Bei Kodierung der schrägen Gefäßwand mit Anti-IgG wird vorzugsweise wie folgt verfahren: 1 ml Anti-IgG Rabbit wird mit 19 ml Carbonat-Bicarbonat-Puffer pH 9,6 vermischt - das entspricht einer Verdünnung von 1: 20. Von dieser Mischung werden jeweils 50 µl pro Kavität einer Spitzboden-Mikrotiterplatte, z.B. einer ELISA-Platten (Fa. Greiner, BRD) einpippetiert und bei Zimmertemperatur über Nacht stehen gelassen. Anschließend wird mit 150 µl PBS-Puffer, pH 7,1, dreimal, zum Beispiel mit PBS-Puffer, gewaschen; Anti-IgG haftet fest an der Wandung. Für die nachfolgende Beschichtung des Gefäßes mit der viskosen Substanz, z.B. Gel aus 61,5 mg Agarose (Typ XIII, low viscosity, Fa. Sigma) pro 100 ml PBS-Puffer pH 7,4, werden 25 µl Gel in jede Kavität einpippetiert, was vorzugsweise bei ca 52 Grad Celsius erfolgt. Eine derartig behandelte Spitz- oder Rundboden-Mikrotiterplatte kann dann bei 4-8 Grad C gelagert werden.

Bei einer weiteren Ausführung erfolgt eine zusätzliche Kodierung der Mikroreaktionsgefäße, vor Beschichtung mit viskoser Substanz, zum Beispiel mit Protein A und/oder Protein G. Dazu wird eine Mischung aus 1 mg Protein A und/oder Protein G pro 100 ml PBS-Puffer, pH 7,1 hergestellt. Von dieser Mischung mit einer Konzentration von 0,01 mg/ml werden jeweils 50 µl pro Kavität einer Spitzboden-Mikrotiterplatte, z.B. ELISA-Platten der Fa. Greiner, BRD, einpippetiert und bei Raumtemperatur über Nacht inkubiert. Anschließend wird dreimal mit PBS-Puffer gewaschen. Anschließend werden die Kavitäten - wie vorstehend bei der zusätzlichen Kodierung mit Anti-IgG beschrieben - mit Gel oder einer anderen viskosen Substanz - beschichtet.

Für die Auflösung des Gels kann anstelle von PBS auch modifizierter Liss-Puffer oder Puffer mit Proteinen verwendet werden.

Zur Kodierung der Gefäßwand können Antikörper oder Proteine wie auch weitere Kodiersubstanzen auf die Wand des Mikroreaktionsgefäßes "geklebt" werden, danach erfolgt die Beschichtung mit der viskosen Substanz.

In einem weiteren Ausführungsbeispiel werden diese beiden Mikrotiterplatten für die Untersuchung auf irrgeuläre Antikörper folgendermassen angesetzt. Aus handelsüblichen Suchzellen von ca. 3 Vol-% wird eine ca. 0,6 Vol-% Gebrauchssuchzellenaufschwemmung wie folgt hergestellt: eine Mischung von 1 Teil 3Vol-% Suchzellenaufschwemmung + 2 Teile modifizerte Lisslösung + 2 Teile modifizierte Bromealinlösung. Das zu untersuchende Serum wird mit modifizierter Lisslösung verdünnt: 1 Teil Serum + 1 Teil Lisslösung. Nunmehr werden 25 µl von dem verdünnten Serum plus 25 µl von der ca. 0,6 Vol-% Gebrauchssuchzellenaufschwemmung vermischt und auf das Gel bzw. die viskose Substanz aufpippetiert. Anschließend wird bei 37 Grad C ca. eine Stunde inkubiert und danach das Ergebnis abgelesen und ausgewertet.

Gewerbliche Anwendbarkeit: Die Erfindung ist insbesondere zum Antikörper-Suchtest, zur Serumgegenprobe, zur erythrozytenseitigen Blutgruppenbestimmung (ABO und RH-Formel), wie auch zur Kreuzprobe geeignet. Mit dem Verfahren kann jede Antigen-Antikörper-Reaktion, die über Partikel bestimmbar ist, durchgeführt werden. Das Verfahren ist insbesondere in Krankenhäusern, in medizinischen Laboratorien oder in Einrichtungen zur Blut- und Plasmagewinnung gewerblich anwendbar. Die besondere Nützlichkeit der Erfindung liegt in der vollautomatischen Durchführbarkeit des Verfahrens.

### Liste der Bezugszeichen:

- 1: Mikroreaktionsgefäß
- 2: viskose substanz oder gelschicht
- 3: Testflüssigkeit oder flüssiges Testgemisch
- 4, 4', 4", 4''': Träger
- 5: Meniskus

## Patentansprüche

1. Verfahren zum Nachweis von Antikörpern und/oder Antigenen in einer Testflüssigkeit sowie zur Blutgruppenbestimmung durch Reaktion mit einem vorgegebenen spezifischen Bindungspartner, wobei das Antigen bzw. der Antikörper oder der spezifische Bindungspartner in der Testflüssigkeit ungebunden oder an einen Träger gebunden ist und wobei bei positiver Antigen-Antikörper-Reaktion ein Agglutinat aus nachzuweisenden Antigenen bzw. Antikörpern, den entsprechenden spezifischen Bindungspartnern und den Trägern gebildet wird, welches optisch als Sedimentationsbild detektierbar ist, **dadurch gekennzeichnet**,
1.1 daß die Reaktion in einem oben offenen und am Boden geschlossenen Mikroreaktionsgefäß durchgeführt wird, das wenigstens in seinem bodennahen Bereich einen sich verjüngenden Querschnitt aufweist, so dass das Gefäß wenigstens in seinem bodennahen Bereich wenigstens teilweise eine schräge Wandung besitzt, die in den Boden übergeht bzw. den Boden bildet,
1.2 mindestens in diesen bodennahen Bereich mit schräger Wandung des Mikroreaktionsgefäßes eine viskose, homogene Substanz, insbesondere erhitzte Gallerte oder erhitztes Gel, eingegossen und erkalten gelassen wird, so dass die Substanz nach dem Erkalten den Bereich des Gefäßes mit schräger Wandung einschließlich des Bodens als dünne, homogene Schicht auf der schrägen Wandung des Gefäßes oder in Form eines Meniskus bedeckt bzw. ausfüllt und dabei eine feine Vernetzung bzw. Gitterstruktur ausbildet;
1.3 ein vorbestimmtes Volumen der Testflüssigkeit in das Gefäß auf die homogene Schicht der viskosen Substanz aufgegeben wird, wobei der spezifische Bindungspartner entweder der Testflüssigkeit oder der viskosen Substanz zugesetzt ist oder eine weitere Flüssigkeit, die den spezifischen Bindungspartner enthält, vor oder nach Zufügen der Testflüssigkeit dem Gefäß zugefügt wird;
1.4 das Mikroreaktionsgefäß wenigstens so lange inkubiert wird, bis sich in der Grenzschicht zwischen der schrägen Wandung des Mikroreaktionsgefäßes und der dünnen, homogenen Schicht aus der viskosen Substanz allein aufgrund der Schwerkraft das sichtbare Sediment/Agglutinat ausbildet, wobei eine insbesondere auf der schrägen Wandung des Gefäßes sich flächig ausbildende Agglutinatschicht auf eine positive Antigen-Antikörper-Reaktion hinweist und ein insbesondere im Bereich des tiefsten Punktes des Bodens bzw. der schrägen Wandung des Gefäßes sich bildende, räumlich gerinfügig ausgedehnte Ablagerung auf eine negative Reaktion ohne Antigen/Antikörper-Komplex hinweist.

2. Verfahren nach Anspruch 1, **dadurch gekennzeichnet,**
**dass** die Querschnittsfläche des Gefäßes im Bereich der Füllhöhe der viskosen Substanz wenigstens das Vierfache der Querschnittsfläche des Gefäßes im Bereich unterhalb der viskosen Substanz beträgt.

3. Verfahren nach Anspruch 1 oder 2, **dadurch gekennzeichnet,**
**dass** der Bereich der schrägen Wandung des Mikroreaktionsgefäßes mit Fangstoffen, nämlich Proteinen, wie Protein A und/oder G, und/oder Antigene und/oder Antikörper, codiert wird, und erst danach die Beschichtung des Mikroreaktionsgefäßes mit der viskosen Substanz erfolgt.

4. Verfahren nach Anspruch 1, **dadurch gekennzeichnet,**
**dass** nach dem Schritt 1.2 inkubiert wird, vorzugsweise bei Temperaturen von wenigstens 5 Grad C, bevorzugt zwischen 10 und 37 Grad C, insbesondere bei Raumtemperatur, und für wenigstens 15 Minuten, bevorzugt wenigstens 50 Minuten.

5. Verfahren nach Anspruch 1, **dadurch gekennzeichnet,**
**dass** einer der Flüssigkeiten bzw. dem Gemisch oder der viskosen Substanz Albumin in einer Konzentration von wenigstens 0,01 mg/ml zugesetzt wird, vorzugsweise zwischen 0,2 mg/ml und 1 mg/ml.

6. Verfahren nach Anspruch 1, **dadurch gekennzeichnet,**
**dass** als viskose Substanz entweder
ein Gel oder eine Gallerte verwendet wird, das bzw. die bis zur Schmelztemperatur erhitzt und in flüssigem Zustand zur Ausbildung der homogenen Schicht in das Mikroreaktionsgefäß eingefüllt wird, wo das Gel bzw. die Gallerte abkühlt und dadurch eine homogene Schicht gebildet wird, wobei das Gel ein organisches Gel, insbesondere Agarose und/oder Gelatine ist und eine Gelkonzentration von wenigstens 0,02 mg/100 ml, bevorzugt im Bereich von 0,1 mg/100 ml bis 6,66 g/100 ml (6666 mg) vorzugsweise zwischen 10 mg/100 ml und 110 mg/100 ml, besonders bevorzugt zwischen 40 mg/100 ml und 80 mg/100 ml aufweist,
oder
die viskose Substanz aus vernetzten Polymeren besteht.

7. Verfahren Anspruch 1, **dadurch gekennzeichnet,**
**dass** im Gel verschiedenste Puffer und/oder spezifische Bindungspartner (Nachweis-Antigene oder Nachweis-Antikörper, insbesondere monoklonale Antikörper) und/oder Träger, enthalten sind, die unter der Voraussetzung der Hitzestabilität im flüssigen Erhitzungsstadium des Gels zugefügt werden.

8. Verfahren nach einem der vorherigen Ansprüche,
**dadurch gekennzeichnet, dass** die Träger innerhalb der Testflüssigkeit Erythrozyten, Leukozyten, Blutplättchen, Latex-Partikel oder Agarose-Partikel, Glaspartikel oder Kunststoffpartikel sind.

9. Verfahren zur Durchführung einer Serumgegenprobe zum Nachweis von Blutgruppen-Antikörpern (irreguläre Antikörper) nach Anspruch 1,
**dadurch gekennzeichnet, dass** die Testflüssigkeit Blutserum enthält, welches auf das Vorhandensein von bestimmten Blutgruppen-Antiköpern untersucht werden soll, und der Testflüssigkeit eine Flüssigkeit zugefügt wird, die Test-Erythrozyten einer bekannten Blutgruppe enthält, welche als Träger mit spezifischen Bindungspartnern für die nachzuweisenden Antikörper bzw. Iso-Agglutinine verbunden sind.

10. Verfahren zur Durchführung einer Blutgruppenbestimmung nach Anspruch 1, **dadurch gekennzeichnet,**
**dass** die Testflüssigkeit, welche auf das Vorhandensein von bestimmten Antigenen untersucht werden soll, Erythrozyten mit unbekannten Antigenen enthält, und der Testflüssigkeit eine Flüssigkeit zugefügt wird, welche bekannte Such-Antikörper enthält.

11. Verfahren nach Anspruch 1, **dadurch gekennzeichnet,**
**dass** einer der Flüssigkeiten bzw. dem Gemisch oder der viskosen Substanz zum Zeitpunkt der Untersuchung Proteine, wie Protein A und/oder G, und/oder Antigene, und/oder Antikörper zugesetzt werden.

12. Verfahren nach einem der vorherigen Ansprüche, **dadurch gekennzeichnet,**
**dass** zur Beschleunigung der Sichtbarmachung der Ausbildung der negativen Reaktion dem Verfahren ein Zentrifugierschritt zugefügt wird.

13. Mikroreaktionsgefäß mit einer Vielzahl von Vertiefungen, zum Nachweis von Antikörpern und/oder Antigenen in einer Testflüssigkeit sowie zur Blutgruppenbestimmung durch Reaktion mit einem vorgegebenen spezifischen Bindungspartner, wobei das Antigen bzw. der Antikörper oder der spezifische Bindungspartner in der Testflüssigkeit ungebunden oder an einen Träger gebunden ist und wobei bei positiver Antigen-Antikörper-Reaktion ein Agglutinat aus nachzuweisenden Antigenen bzw. Antikörpern, den entsprechenden spezifischen Bindungspartnern und den Trägern gebildet wird, welches optisch als Sedimentationsbild detektierbar ist,
**dadurch gekennzeichnet, daß** das Mikroreaktionsgefäß oben offen und am Boden geschlossen ist und wenigstens in seinem bodennahen Bereich einen sich verjüngenden Querschnitt aufweist, so dass das Gefäß wenigstens in seinem bodennahen Bereich wenigstens teilweise eine schräge Wandung besitzt, die in den Boden übergeht bzw. den Boden bildet, wobei sich mindestens in diesem bodennahen Bereich mit schräger Wandung des Mikroreaktionsgefäßes eine viskose Substanz, vorzugsweise Gel oder Gallerte, befindet, welche darin den Bereich des Gefäßes mit schräger Wandung einschließlich des Bodens als homogene Schicht oder in Form eines Meniskus bedeckt bzw. ausfüllt und dabei eine feine Vernetzung bzw. Gitterstruktur ausbildet zum Aufbringen eines vorbestimmten Volumes der Testflüssigkeit auf die viskose Substanz.

14. Verwendung von Spitzboden- oder Rundboden-Mikrotiterplatten mit einer Vielzahl von V-förmigen oder bombierten Vertiefungen, in welchen eine viskose Substanz in Form einer dünnen, homogenen Schicht oder eines Meniskus wenigstens im Bereich der schräger Wandung enthalten ist unter Ausbildung einer Grenzschicht zwischen der schrägen Wandung des Mikroreaktionsgefäßes und der homogenen Schicht aus der viskosen Substanz zur Durchführung eines Tests zum Nachweis von Antikörpern oder Antigenen durch Detektion einer Agglutinationsreaktion oder zur Blutgruppenbestimmung.

15. Verwendung gemäß Anspruch 14, **dadurch gekennzeichnet, dass** wenigstens im Bereich der schrägen Wandung Fangstoffe, nämlich Proteine, wie Protein A und/ oder G, und/oder Antigene, und/oder Antikörper als Kodierung aufgebracht sind.

16. Verwendung einer viskosen Substanz in einem Mikroreaktionsgefäß mit einer V-förmigen oder bombierten Vertiefung mit schräger Wandung zur Ausbildung einer dünnen, homogenen Schicht aus der viskosen Substanz wenigstens im Bereich der schrägen Wandung zur Durchführung eines Tests zum Nachweis von Antikörpern oder Antigenen oder zur Blutgruppenbestimmung durch Detektion einer Agglutinationsreaktion, wobei als viskose Substanz
entweder
ein Gel oder eine Gallerte verwendet wird, wobei das Gel ein organisches Gel, insbesondere Agarose und/oder Gelatine ist und eine Gelkonzentration von wenigstens 0,02 mg/100 ml, bevorzugt im Bereich von 0,1 mg/100 ml bis 6,66 g/100 ml (6666 mg) vorzugsweise zwischen 10 mg/100 ml und 110 mg/100 ml, besonders bevorzugt zwischen 40 mg/100 ml und 80 mg/100 ml aufweist,
oder
die viskose Substanz aus vernetzten Polymeren besteht.

## Claims

1. Method for detecting of antibodies and/or antigen in a test liquid as well as for blood group determination by reaction with a predetermined specific binding partner, wherein the antigen or, respectively, the antibody or the specific binding partner in the test liquid is unbound or bound to the carrier and wherein upon a positive antigen antibody reaction an agglutinate of antigen or, respectively, antibodies, the corresponding specific binding partners and the carriers is formed, which is optically detectable as a sedimentation picture, **characterized in that**
1.1. The reaction is performed in a micro-reaction vessel open on the top and closed at the bottom, wherein the micro-reaction vessel exhibits at least in its region close to the floor a narrowing down cross-section, such that the vessel has at least partly inclined wall at least in the region close to the floor, which inclined wall transitions into the floor or, respectively forms the floor,
1. 2. The viscous homogeneous substance, in particular a heated jelly or a heated gel cast and allowed to cool down at least in this floor close region with inclined wall of the vessel, such that the substance covers or, respectively, fills after the cooling down the region of the vessel with inclined wall including the floor as a homogeneous layer and thereby forms a cross linking or, respectively, a grid structure;
1. 3.A predetermined volume of the test liquid is added onto the viscous substance the vessel, wherein the specific binding partner is added either to the test liquid or to the viscous substance, or a further liquid, which contains the specific binding partner, is added prior to or after adding of the test liquid to the vessel;
1.4. The micro-reaction vessel is incubated for at least such a long time until the visible sediment/agglutinate forms out of the viscous substance alone based on gravity force in the boundary layer between the inclined wall of the micro-reaction vessel and the thin homogeneous layer out of the viscous substance, wherein in particular a flat formed agglutinate layer on the inclined wall of the vessel points to a positive antigen anti-body reaction and wherein a spatially slightly extended deposit formed in particular in the region of the lowest point of the floor or, respectively, inclined wall of the vessel points to a negative reaction without antigen/antibody complex.

2. Method according to claim 1, **characterized in that** the cross-section of face of the vessel in the region of the filling height level of the viscous substance amounts to at least a fourfold of the cross-section of face of the vessel in the region below the viscous substance.

3. Method according to claims 1 or 2 **characterized in that**
the region of the inclined wall of the micro-reaction vessel is coded with capture materials, namely proteins, as protein A and/or G, and/or antigens and/or antibodies and only thereupon the coating of the micro-reaction vessel with the viscous substance is performed.

4. Method according to claim 1 **characterized in that**
incubation is performed according to the step 1.2, preferably at temperatures from at least five degrees centigrade, preferably between 10 and 37 degrees centigrade, in particular room temperature and for at least 15 minutes, preferably at least 50 minutes.

5. Method according to claim 17, **characterized in that**
albumin in the concentration of at least 0.01 mg per ml and preferably between 0.2 mg per ml and 1 mg per ml is added to one of the liquids or, respectively, the viscous substance.

6. Method according claim 1 **characterized in that**
the viscous homogeneous substance, in particular a heated jelly or a heated gel cast is allowed to cool down at least in this floor close region with inclined wall of the vessel, such that the substance covers or, respectively, fills after the cooling down the region of the vessel with inclined wall including the floor as a homogeneous layer. The gel is an organic gel, in particular either agarose and/or gelatin and exhibits a gel concentration of at least 0.02 mg per hundred milliliters, preferably in the region of the 0.1 mg per hundred milliliters to 6.66 grams per hundred milliliters (6666 mg) preferably between 10 mg per hundred milliliters and under 110 mg per hundred milliliters, particularly preferred between 40 mg per hundred milliliters and 80 mg per hundred milliliters,
or
the viscous substance is formed of cross linked polymers.

7. Method according to claim 1 **characterized in that**
various buffers and/or specific binding partners (detecting antigen or detecting anti-body, in particular monoclonal antibodies) and/or carriers are contained in the gel, which are added in a liquid heating state of the gel under the precondition of heat stability.

8. Method according to one of the preceding claims **characterized in that**
the carriers within the test liquid are erythrocytes, leucocytes, blood platelets, latex particles or agarose particles, glass particles or plastic particles.

9. Method for performing of a serum counter test for detecting of blood group antibodies (irregular antibodies) according claim 1 **characterized in that**
the test liquid contains blood serum, which is to be investigated relative to the presence of certain blood group antibodies, and wherein a liquid is added to the test liquid, wherein the liquid contains test erythrocytes of a known blood group, which are connected as carriers with specific binding partners for the antibodies or, respectively, iso-agglutinins to be detected.

10. Method for performing of blood group determination according claim 1 **characterized in that**
the test liquid, which is to be investigated relative to the presence of certain antigens contained erythrocytes with unknown antigen and wherein a liquid is added to the test liquid, which liquid contains known search and find antibodies.

11. Method according to claim 14 or 15 **characterized in that**
proteins as protein A and/or protein G and/or antigens and/or antibodies are added to one of the liquids or, respectively, the mixture or to the viscous substance at the point in time of the investigation.

12. Method according to one of the preceding claims **characterized in that**
a centrifuging step is added to the process to speed up the visibility of a negative reaction.

13. A micro-reaction vessel with a plurality of recesses for detecting of antibodies and/or antigen in a test liquid as well as for blood group determination by reaction with a predetermined specific binding partner, wherein the antigen or, respectively, the antibody or the specific binding partner in the test liquid is unbound or bound to the carrier and wherein upon a positive antigen antibody reaction an agglutinate of antigen or, respectively, antibodies, the corresponding specific binding partners and the carriers is formed, which is optically detectable as a sedimentation picture **characterized in that**
the reaction is performed in a micro-reaction vessel open on the top and closed at the bottom, wherein the micro-reaction vessel exhibits at least in its region close to the floor a narrowing down cross-section, such that the vessel has at least partly inclined wall at least in the region close to the floor, which inclined wall transitions into the floor or, respectively forms the floor whereby a viscous homogeneous substance, in particular a jelly or a gel is applied at least in this region close to the floor with inclined wall of the vessel, such that the substance covers or, respectively, fills the region of the vessel with inclined wall including the floor as a homogeneous layer and thereby forms a cross linking or, respectively, a grid structure;

14. Use of tipped floor or round floor microtitration plates with a plurality of V-shaped or bombed recesses, wherein a viscous substance in the form of a thin homogeneous or meniscal layer is contained in the recesses of the micro reaction vessels at least in the region of the V-shaped or bombed recesses under formation of a boundary layer between the inclined wall of the micro-reaction vessel and the homogeneous layer out of the viscous substance for performing a test for detecting of antibodies or antigens or for blood group determination by detection of an agglutination reaction.

15. Use according to claim 14 **characterized in that**
capturing materials, namely protein, as protein A and/or protein G and/or antigens and/or antibodies are applied in the plurality of recesses at least in the region of the V-shaped or bombed recesses as a coding.

16. Use of a viscous substance in tipped floor or round floor microtitration plates wherein this substance is contained in the form of a homogeneous layer at least in the region of the V-shaped or bombed recess under formation of a boundary layer between the inclined wall of the micro-reaction vessel and the homogeneous layer out of the viscous substance for performing a test for detecting of antibodies or antigens by detecting and agglutination reaction or for blood group determination whereby this substance can be
either
a gel as an organic gel, in particular either agarose and/or gelatin, exhibiting a gel concentration of at least 0.02 mg per hundred milliliters, preferably in the region of the 0.1 mg per hundred milliliters to 6.66 grams per hundred milliliters (6666 mg) preferably between 10 mg per hundred milliliters and under 110 mg per hundred milliliters, particularly preferred between 40 mg per hundred milliliters and 80 mg per hundred milliliters,
or
that the viscous substance is formed of cross linked polymers.

## Revendications

1. Procédé pour mettre en évidence la présence d'anticorps et/ou d'antigènes dans un liquide d'essai ainsi que pour déterminer des groupes sanguins par la réaction avec un partenaire de liaison spécifique prédéfini, l'antigène ou l'anticorps ou le partenaire de liaison spécifique étant non lié ou lié à un support dans le liquide d'essai, et, lorsque la réaction antigène-anticorps est positive, un agglutinat composé d'antigènes ou d'anticorps à déceler, de partenaires de liaison spécifiques et de supports se formant lequel peut être détecté optiquement comme image de sédimentation, **caractérisé en ce que**
1.1 la réaction s'effectue dans un réacteur ouvert vers le haut et à fond fermé et dont la section diminue au moins dans la zone voisine du fond, de sorte que le réacteur présente au moins dans la zone voisine du fond au moins en partie une paroi inclinée s'étendant jusque dans le fond ou formant le fond,
1.2 au moins dans cette zone voisine du fond et à paroi inclinée du microréacteur, une substance homogène visqueuse, en particulier de la gelée ou du gel chauffés, est coulée qui, après refroidissement, recouvre ou remplit la zone du réacteur à paroi inclinée en forme de couche mince homogène sur la paroi inclinée du réacteur ou en forme de ménisque tout en conformant une structure fine de réseau ou de grille;
1.3 un volume prédéterminé du liquide d'essai est versé dans le réacteur sur la couche homogène de la substance visqueuse, le partenaire de liaison spécifique étant ajouté ou au liquide d'essai ou à la substance visqueuse, ou bien un autre liquide contenant le partenaire de liaison spécifique étant versé dans le réacteur avant ou après l'introduction du liquide d'essai;
1.4 le microréacteur est incubé au moins jusqu'à ce que le sédiment/l'agglutinat visible se forme uniquement par gravité dans la couche limite entre la paroi inclinée du microréacteur et la couche mince homogène constituée par la substance visqueuse, une couche d'agglutinat en nappe se formant en particulier sur la paroi inclinée du réacteur indiquant une réaction antigène-anticorps positive et un dépôt de faible étendue dans l'espace se formant en particulier dans la zone du point le plus bas du fond ou de la paroi inclinée du réacteur indiquant une réaction négative sans complexe antigène-anticorps.

2. Procédé selon la revendication 1, **caractérisé en ce que**
l'aire de la section transversale du réacteur dans la zone du niveau de remplissage de la substance visqueuse s'élève au moins au quadruple de l'aire de la section transversale du réacteur dans la zone en dessous de la substance visqueuse.

3. Procédé selon la revendication 1 ou 2, **caractérisé en ce que**
la zone de la paroi inclinée du microréacteur est codée par des substances de captation, à savoir des protéines tels la protéine A et/ou G et/ou des antigènes et/ou des anticorps et que le revêtement du microréacteur par la substance visqueuse ne s'effectue que par la suite.

4. Procédé selon la revendication 1, **caractérisé en ce que**
l'incubation a lieu après l'étape 1.2, de préférence à des températures d'au moins 5°C, de façon plus préférentiellement entre 10 et 37°C, en particulier à la température ambiante, et pendant au moins 15 minutes, de préférence au moins 50 minutes.

5. Procédé selon la revendication 1, **caractérisé en ce que**
l'on ajoute à l'un des liquides ou au mélange ou à la substance visqueuse de l'albumine à une concentration d'au moins 0,01 mg/ml, de préférence entre 0,2 mg/ml et 1 mg/ml.

6. Procédé selon la revendication 1, **caractérisé en ce que**
la substance visqueuse utilisée est
ou un gel ou une gelée qui est chauffé à la température de fusion et introduit à l'état liquide dans le microréacteur pour former la couche homogène où le gel ou la gelée se refroidit en formant une couche homogène, le gel étant un gel organique, en particulier de l'agarose et/ou de la gélatine présentant une concentration en gel d'au moins 0,02 mg/100 ml, de préférence dans une plage de 0,1 mg/100 ml à 6,66 g/100 ml (6666 mg), de façon préférentielle entre 10 mg/100 ml et 110 mg/ml, et de façon toute préférentielle entre 40 mg/100 ml et 80 mg/100 ml,
ou la substance visqueuse consiste en polymères réticulés.

7. Procédé selon la revendication 1, **caractérisé en ce que**
le gel contient différents tampons et/ou partenaires de liaison (antigènes traceurs ou anticorps traceurs, en particulier anticorps monoclonaux) et/ou supports, qui, à condition d'être stables à la chaleur, sont ajoutés au gel chauffé à l'état liquide.

8. Procédé selon l'une quelconque des revendications précédentes, **caractérisé en ce que**
les supports dans le liquide d'essai sont des érythrocytes, des leucocytes, des thrombocytes, des particules de latex, des particules d'agarose, des particules de verre ou des particules de matière plastique.

9. Procédé pour effectuer une contre-épreuve sérique afin de mettre en évidence des anticorps de groupe sanguin (anticorps irréguliers) selon la revendication 1, **caractérisé en ce que**
le liquide d'essai contient du sérum sanguin qui doit être examiné en vue de déterminer la présence d'anticorps de groupe sanguin déterminés et qu'on ajoute au liquide d'essai un liquide contenant des érythrocytes d'essai d'un groupe sanguin connu qui, en tant que supports, sont reliés à des partenaires de liaison spécifiques pour les anticorps à mettre en évidence ou iso-agglutinines.

10. Procédé pour effectuer la détermination du groupe sanguin selon la revendication 1, **caractérisé en ce que**
le liquide d'essai qui doit être examiné pour déterminer la présence d'antigènes déterminés, contient des érythrocytes avec des antigènes inconnus et qu'on ajoute au liquide d'essai un liquide contenant des anticorps de recherche connus.

11. Procédé selon la revendication 1, **caractérisé en ce que**,
au moment de l'analyse, des protéines, telles de la protéine A et/ou G et/ou des antigènes et/ou des anticorps sont ajoutés à l'un des liquides ou au mélange ou à la substance visqueuse.

12. Procédé selon l'une quelconque des revendications précédentes, **caractérisé en ce que**,
une étape de centrifugation est ajoutée au procédé afin d'accélérer la visualisation de la formation de la réaction négative.

13. Microréacteur comprenant une pluralité de cavités pour mettre en évidence des anticorps et/ou des antigènes dans un liquide d'essai et pour déterminer des groupes sanguins par la réaction avec un partenaire de liaison spécifique prédéfini, l'antigène ou l'anticorps ou le partenaire de liaison spécifique étant non lié ou lié à un support dans le liquide d'essai, et, lorsque la réaction antigène-anticorps est positive, un agglutinat composé d'antigènes ou d'anticorps à déceler, de partenaires de liaison spécifiques correspondants et de supports se formant, lequel peut être détecté optiquement en tant qu'image de sédimentation, **caractérisé en ce que**
le microréacteur est ouvert vers le haut et à fond fermé et présente une section diminué au moins dans la zone voisine du fond et que le réacteur possède, au moins dans la zone voisine du fond, au moins partiellement une paroi inclinée qui s'étend jusque dans le fond ou qui forme le fond, une substance visqueuse, de préférence du gel ou de la gelée, se trouvant au moins dans cette zone voisine du fond à paroi inclinée du microréacteur, laquelle recouvre ou remplit en forme de couche homogène ou de ménisque la zone à paroi inclinée y compris le fond du réacteur tout en formant une structure fine de réseau ou de grille pour l'application d'un volume prédéterminé du liquide d'essai sur la substance visqueuse.

14. Utilisation de plaques microtitre à fond pointu ou à fond rond comprenant une pluralité de cavités en V ou bombées, qui contiennent une substance visqueuse sous forme de couche mince homogène ou de ménisque au moins dans la zone de la paroi inclinée tout en formant une couche limite entre la paroi inclinée du microréacteur et la couche homogène de la substance visqueuse, pour effectuer un test de mise en évidence d'anticorps ou d'antigènes par détection d'une réaction d'agglutination ou pour déterminer des groupes sanguins.

15. Utilisation selon la revendication 14, **caractérisée en ce que**
des substances de captation, à savoir des protéines telles la protéine A et/ou G, et/ou des antigènes et/ou des anticorps sont appliqués comme codage au moins dans la zone de la paroi inclinée.

16. Utilisation d'une substance visqueuse dans un microréacteur à cavité en V ou bombée avec une paroi inclinée pour former une couche mince homogène de ladite substance visqueuse au moins dans la zone de la paroi inclinée afin d'effectuer un test pour la mise en évidence d'anticorps ou d'antigènes ou pour la détermination de groupes sanguins par détection d'une réaction d'agglutination, la substance visqueuse utilisée étant
ou un gel ou une gelée, le gel étant un gel organique, en particulier de l'agarose et/ou de la gélatine présentant une concentration en gel d'au moins 0,02 mg/100 ml, de préférence dans une plage de 0,1 mg/100 ml à 6,66 g/100 ml (6666 mg), de façon préférentielle entre 10 mg/100 ml et 110 mg/ml, et de façon toute préférentielle entre 40 mg/100 ml et 80 mg/100 ml,
ou la substance visqueuse consistant en polymères réticulés.
